# EUROPEAN PATENT APPLICATION

(11) **EP 4 454 542 A1**
(43) Date of publication of application: **30.10.2024**
(21) Application number: 22913767.4
(22) Date of filing: 31.10.2022
(51) Int. Cl.: A61B 1/07, A61B 1/04, G02B 23/24

(54) **ENDOSCOPE PHOTOGRAPHIC SYSTEM, ENDOSCOPE APPARATUS, AND ENDOSCOPE DATA TRANSMISSION CABLE**

(30) Priority: 30.12.2021 WO PCT/CN2021/143064; 31.12.2021 CN 202111672585
(71) Applicant: Wuhan Mindray Bio-Medical Scientific Co., Ltd., Wuhan City Hubei 430206 (CN); Shenzhen Mindray Bio-Medical Electronics Co., Ltd., Shenzhen, Guangdong 518057 (CN)
(72) Inventor: SHE, Yanchao, Shenzhen, Guangdong 518057 (CN); LIU, Bin, Shenzhen, Guangdong 518057 (CN); CHEN, Dabing, Shenzhen, Guangdong 518057 (CN); JIAO, Kun, Shenzhen, Guangdong 518057 (CN); FU, Xing, Shenzhen, Guangdong 518057 (CN)
(74) Representative: KIPA AB
(86) International application number: PCT/CN2022/128815
(87) International publication number: WO 2023/124504

(57) **Abstract**

An endoscope data transmission cable (71). "Electro-optical-electro" conversion is realized at two ends of the endoscope data transmission cable (71), respectively. A high-speed video signal in the cable (71) is transmitted by using an optical fiber (204), thereby ensuring the signal transmission quality and reliability. In addition, an electro-optical conversion module and a photoelectric conversion module are integrated at two ends of the cable (71), an electrical signal at a camera (40) end is transmitted to the cable (71) by means of an electrical connector (205), and is then converted into an optical signal by means of the electro-optical conversion module, the optical signal is transmitted in the cable (71) by means of the optical fiber (204), and is then converted, at a connection end of the cable (71) and an image processing device (50), into the electrical signal by means of the photoelectric conversion module, and the electrical signal is then transmitted into the image processing device (50) by means of an electrical connector.

## Description

### TECHNICAL FIELD

The disclosure relates to a medical diagnostic device, and more particularly to an endoscope imaging system, an endoscope apparatus, and an endoscope data transmission cable.

### BACKGROUND

With improvement of technology, a requirement for image definition of endoscope imaging system becomes higher and higher, and data volume processed by an imaging head of the endoscope imaging system is larger and larger. As data inside the imaging head need to be reliably transmitted to an image processing device for processing, and then outputted to a display for displaying, a higher requirement is provided for long-distance transmission of high-speed data.

At present, a cable for the imaging head is generally 3-6 m in clinical practice. Video acquisition of 4K definition is currently employed, while video acquisition of 8K definition or even higher will be employed in the future. Meanwhile, more than one image sensor may be provided inside the imaging head. In such high-speed case, most common cables cannot satisfy this high-speed transmission requirement, even for some cables which are capable of transmitting these high-speed signals, signal attenuation is also exceptionally large, and an interface chip is needed for processing these high-speed signals, but the reliability of these high-speed signals is still difficult to satisfy the requirement. Since a power supply, low-speed communication signals and high-speed communication signals, are included between the imaging head and the image processing device, an active cable solution can effectively solve various problems of high-speed communication between the endoscope imaging head and an imaging head host by the current cable, in combination with the cost and the manufacturability angle. The active cable refers to a communication cable for converting an electrical signal into an optical signal or converting an optical signal into an electrical signal during communication.

In a general solution, a pure cable is used between the endoscope imaging head and the image processing device for data transmission, a universal high-speed electrical connector is used to connect a cable plug and a socket of the image processing device, and the image processing device processes attenuated high-speed signals through an interface chip. The disadvantage of this solution is that high-speed signals attenuate greatly after long-distance cable transmission, making them susceptible to interference. Moreover, it is difficult to ensure a reliability of transmitting high-speed video data to the host end even through the interface chip processing. In addition, pure cables are relatively thick, heavy overall, and have poor flexibility. As the cable is connected with a handle of the imaging head, which handle is operated by the user, it seriously affects the operation experience of the user.

In some other solutions, a photo-electric composite cable is used between the endoscope imaging head and the image processing device for data transmission, a high-speed video signal is transmitted by using lenses and mirrors between the cable plug and the socket of the image processing device. The cable plug and the socket of the image processing device belong to an optical connector (optical connector solution). Then, the optical signal is converted into an electrical signal in the image processing device. Since the cable plug and the socket of the image processing device can be repeatedly plugged into and pulled out of by the user, a tolerance requirement for assembling optical components, such as lenses and mirrors, is relatively high, so that the cost of the mechanical structure is increased. In addition, the optical assembly has a dirty risk, so that the reliability of data transmission is reduced, and signal transmission errors are easily caused.

### SUMMARY

According to a first aspect of this disclosure, an embodiment of this disclosure provides an endoscope apparatus, including:
an operation portion, which includes a housing with a shape that is capable of being gripped, and an image processing component which is arranged inside the housing; wherein the image processing component is configured to at least output an image signal of a region of a patient to be observed; a proximal end of the operation portion is provided with a first interface;
a data transmission cable, which includes a second interface and a first optical module which are arranged at a distal end of the data transmission cable; wherein the second interface is connected with the first interface; the first optical module is electrically connected with the image processing component, and is configured to obtain a first electrical signal from the image processing component, and convert the first electrical signal into a first optical signal; wherein the first electrical signal at least includes the image signal;
the data transmission cable further includes at least one optical fibre, wherein a distal end of the optical fibre is connected with the first optical module, the optical fibre is configured to transmit the first optical signal which is generated by the first optical module to a proximal end of the optical fibre;
the data transmission cable further includes an electrical connector and a second optical module, which are arranged at a proximal end of the data transmission cable; wherein the second optical module is connected with the proximal end of the optical fibre, and is configured to obtain the first optical signal which is transmitted by the optical fibre, and convert the first optical signal into a second electrical signal; wherein the second electrical signal at least includes the image signal; wherein the electrical connector is configured to be pluggable to an image processing device, so as to transmit the second electrical signal to the image processing device.

According to a second aspect of this disclosure, an embodiment of this disclosure provides another endoscope apparatus, including:
an operation portion, which includes a housing with a shape that is capable of being gripped, and an image processing component which is arranged inside the housing; wherein the image processing component is configured to at least output an image signal of a region of a patient to be observed; a proximal end of the operation portion is provided with a first interface;
the operation portion further includes a first optical module, the first optical module is electrically connected with the image processing component, and is configured to obtain a first electrical signal from the image processing component, and convert the first electrical signal into a first optical signal, wherein the first electrical signal at least includes the image signal; and
a data transmission cable, which includes a second interface that is arranged at a distal end of the data transmission cable, wherein the second interface is connected with the first interface;
the data transmission cable further includes at least one optical fibre, wherein a distal end of the optical fibre is connected with the first optical module through an optical connector, wherein the optical fibre is configured to obtain the first optical signal from the first optical module and transmit the first optical signal to a proximal end of the optical fibre;
the data transmission cable further includes an electrical connector and a second optical module, which are arranged at a proximal end of the data transmission cable; wherein the second optical module is connected with the proximal end of the optical fibre, and is configured to obtain the first optical signal which is transmitted by the optical fibre, and convert the first optical signal into a second electrical signal; wherein the second electrical signal at least includes the image signal; wherein the electrical connector is configured to be pluggable to an image processing device, so as to transmit the second electrical signal to the image processing device.

According to a third aspect of this disclosure, an embodiment of this disclosure provides an endoscope data transmission cable, including a second interface and a first optical module which are arranged at a distal end of the endoscope data transmission cable; wherein the second interface is connected with a first interface of an operation portion of an endoscope apparatus, the first optical module is electrically connected with an image processing component of the operation portion, so as to obtain a first electrical signal from the image processing component, and convert the first electrical signal into a first optical signal; wherein the first electrical signal at least includes an image signal of a region of a patient to be observed, which signal is outputted by the image processing component;
the endoscope data transmission cable further includes at least one optical fibre, wherein a distal end of the optical fibre is connected with the first optical module; wherein the optical fibre is configured to transmit the first optical signal which is generated by the first optical module to a proximal end of the optical fibre;
the endoscope data transmission cable further includes an electrical connector and a second optical module, which are arranged at a proximal end of the endoscope data transmission cable; wherein the second optical module is connected with the proximal end of the optical fibre, and is configured to obtain the first optical signal which is transmitted by the optical fibre, and convert the first optical signal into a second electrical signal; wherein the second electrical signal at least includes the image signal; and the electrical connector is configured to be pluggable to an image processing device, so as to transmit the second electrical signal to the image processing device.

According to a fourth aspect of this disclosure, an embodiment of this disclosure provides an endoscope imaging system, including a light source, a light guide beam, an image processing device, and any one of the above endoscope apparatuses, wherein the light source is connected with the endoscope apparatus through the light guide beam.

### BRIEF DESCRIPTION OF THE DRAWINGS

FIG. 1 is a structural diagram of an endoscope imaging system according to an embodiment of this disclosure.
FIG. 2 is a structural diagram of an endoscope apparatus according to an embodiment of this disclosure.
FIG. 3 is a schematic diagram of an internal structure of an operation portion according to an embodiment of this disclosure.
FIG. 4 is a structural diagram of a distal end of a data transmission cable according to an embodiment of this disclosure.
FIG. 5 is a structural diagram of a distal end of a data transmission cable according to an embodiment of this disclosure.
FIG. 6 is a structural diagram of a proximal end (an electrical connector) of a data transmission cable according to an embodiment of this disclosure.
FIG. 7 is a structural diagram of an electrical connector of a data transmission cable according to an embodiment of this disclosure.
FIG. 8 is a structural diagram of a wire spring pin and a wire spring jack according to an embodiment of this disclosure.
FIG. 9 is a schematic diagram of an internal structure of a data transmission cable according to an embodiment of this disclosure.
FIG. 10 is a structural diagram of a spiral tube in a data transmission cable according to an embodiment of this disclosure.
FIG. 11 is a structural diagram of a data transmission cable according to an embodiment of this disclosure.
FIG. 12 is a structural diagram of a data transmission cable according to an embodiment of this disclosure.
FIG. 13 is a structural diagram of an endoscope apparatus according to an embodiment of this disclosure.

### DESCRIPTION OF THE PREFERRED EMBODIMENTS

In order to make technical problems, technical solutions and beneficial effects to be solved by this disclosure clearer, this disclosure is further described in detail below with reference to the accompanying drawings and embodiments. It should be understood that the specific embodiments described herein are only used to explain this disclosure and are not intended to limit this disclosure.

In the following description, when referring "some embodiments," it describe a subset of all possible embodiments, but it is understood that "some embodiments" may be the same subset or different subsets of all possible embodiments, and may be combined with each other without conflict.

Unless otherwise defined, all technical and scientific terms used herein have the same meaning as commonly understood by one of ordinary skill in the art to which this disclosure belongs. The technical terms used herein are for the purpose of describing the embodiments of this disclosure only and is not intended to limit this disclosure.

The terms "first", "second", etc., in this disclosure are operable to distinguish different objects, rather than to describe a specific order. In addition, the terms "connection", and "linkage" mentioned in this disclosure include direct and indirect connection (linkage), unless otherwise specified.

When requiring implementing optical transmission of an image signal, it is necessary to convert the image signal into an optical signal at an imaging head end through an electro-optic conversion module, and after the optical signal is transmitted through the optical fibre, the optical signal is converted into the electrical signal through a photo-electric conversion module for processing by the image processing device. The electro-optic conversion module and the photo-electric conversion module both have a certain volume, and the electro-optic conversion module is arranged at one end of a cable connected with the imaging head, which usually can properly share a space inside the imaging head body, or even the electro-optic conversion module is as a part of the imaging head, and is directly arranged inside the imaging head. Therefore, the design of the electro-optic conversion module on the volume of the cable head end is not greatly affected. However, the photo-electric conversion module is located at one end of the cable connected with the image processing device, and the design herein needs to take into account a size of a head end of the cable and a spatial layout of the photo-electric conversion module, which has a certain design difficulty for a person skilled in the art. This is also one of the factors why the aforementioned optical connector solution is employed. In the optical connector solution, the cable is connected with the image processing device through the optical connector, and the photo-electric conversion module is arranged inside the image processing device and has enough space for layout, so that the problem does not exist. Certainly, the optical connector solution also brings a new problem of high cost and low reliability mentioned above.

This disclosure proposes to realize "electro-optic-electric" conversion at both ends of an endoscope data transmission cable for the first time. A high-speed video signal in the cable is transmitted by using an optical fibre to ensure quality and reliability of signal transmission. Meanwhile, the electro-optic conversion module and the photo-electric conversion module are integrated at both ends of the cable, an electrical signal at the imaging head end is transmitted to the cable through the electrical connector, and is converted into an optical signal through the electro-optic conversion module, the converted optical signal is transmitted in the cable through the optical fibre, and the converted optical signal is further converted into the electrical signal through the photo-electric conversion module at an end of the cable, which end is connected with the image processing device, and then transmitted to an interior of the image processing device through the electrical connector.

In the embodiments of this disclosure, the image signal, as a high-speed signal, employes optical fibre transmission, and electro-optic-electric conversion is realized on the data transmission cable, in such a way, long-distance communication is supported, and meanwhile, a signal quality transmitted to the image processing device is ensured, and the optical fibre can also reduce its diameter and weight, thus improving a hand feeling of the user.

The photo-electric conversion module and the electro-optic conversion module are respectively integrated at two ends of the data transmission cable, so that special requirements, such as part tolerance and lens cleanliness when the photo-electric conversion module is integrated at the end of the image processing device, are avoided, the cost is reduced, while the signal quality is ensured and the reliability is improved.

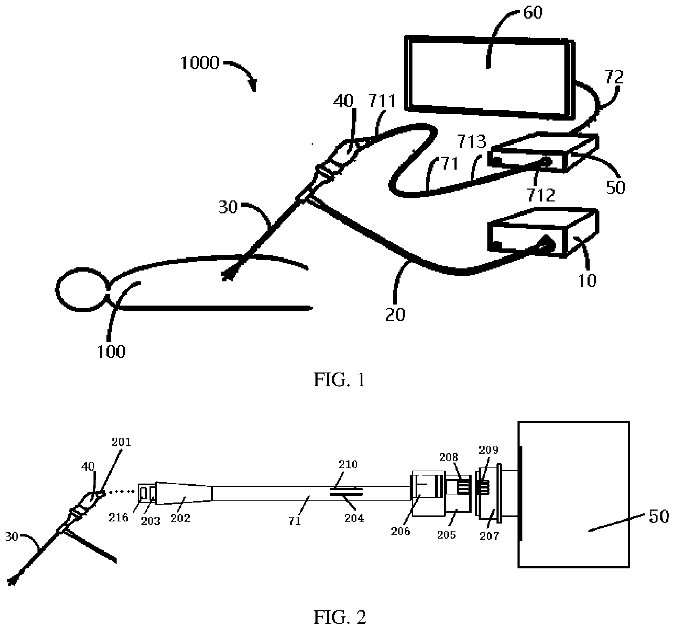

As shown in FIG. 1, an embodiment of this disclosure provides an endoscope imaging system 1000, including a light source 10, a light guide beam 20, an endoscope imaging head 40, a data transmission cable 71, an image processing device 50, a display 60, and a video connection line 72. A endoscope 30 is configured to be inserted into a region of a patient 100 to be observed, and includes an illumination light path and an imaging light path

The image processing device 50 is connected with the endoscope imaging head 40 through the data transmission cable 71, and an image signal generated by the endoscope imaging head 40 is transmitted to the image processing device 50 through the data transmission cable 71 for processing.

In an embodiment, the endoscope imaging head 40 converts an image signal (electrical signal) into an optical signal and transmits the converted optical signal to the image processing device 50 by the data transmission cable 71, and the image processing device 50 converts the converted optical signal into the electrical signal (the image signal). In another embodiment, one end of the data transmission cable 71, which end is connected with the imaging head 40, is provided with an electro-optic conversion module for converting the image signal into an optical signal; and one end of the signal transmission cable 71, which end is connected with the image processing device 50 is provided with a photo-electric conversion module for converting the transmitted optical signal back into the electrical signal and transmitting same to the image processing device 50

The image processing device 50 is connected with a display 60 through the video connection line 72 for transmitting the image signal to the display 60 for displaying. In some embodiments, the data transmission cable 71 may be an optical communication cable, such as an optical fibre; in another embodiment, the data transmission cable 71 may also be a photo-electric composite communication cable.

In an embodiment, the endoscope 30 may be an optical hard endoscope, and the imaging head 40 is provided with one or more image sensors for converting an image light transmitted by the optical hard endoscope into an image signal. The optical hard endoscope is in connection with the imaging head 40 through a snap-fit structure, which connection is capable of being disconnected.

In another embodiment, the endoscope 30 is also a hard endoscope, but the image sensor is arranged at a head end of the endoscope 30, which end is inserted into the patient 100, the image sensor converts the image light acquired by the head end of the endoscope 30 into an image signal and then transmits the image signal to the imaging head 40, and the imaging head 40 is internally provided with an image processing component for processing the image signal. The endoscope 30 is integrated with the imaging head 40, and the endoscope 30 and the imaging head 40 are not detachable during operation. Typical 3D hard endoscope is of this configuration.

In another embodiment, the endoscope 30 may be a flexible structure, the image sensor is arranged at a head end of the endoscope 30, which end is inserted into the patient 100, the image sensor converts the image light acquired by the head end of the endoscope 30 into an image signal and then transmits the image signal to the imaging head 40, and the imaging head 40 is internally provided with an image processing component for processing the image signal. The endoscope 30 is integrated with the imaging head 40, and the endoscope 30 and the imaging head 40 are not detachable during operation. Typical electron soft endoscope is of this configuration.

It should be understood by those skilled in the art that, FIG. 1 is only an example of the endoscope imaging system 1000, and does not constitute a limitation on the endoscope imaging system 1000, and the endoscope imaging system 1000 may include more or fewer components than those shown in FIG. 1, or combine some components, or have different components.

Referring to FIGS. 2-9, an embodiment of this disclosure provides an endoscope apparatus, including an operation portion 40 and a data transmission cable 71.

The operation portion 40 is also referred to as an imaging head (i.e., 40 in FIG. 1). The operation portion 40 includes a housing with a shape which is capable of being gripped, and an image processing component 80 which is arranged inside the housing. The image processing component 80 is configured to at least output an image signal of a region to be observed of the patient, and a proximal end of the operation portion 40 is provided with a first interface 201.

The data transmission cable 71 includes a second interface 202 and a first optical module 203 which are arranged at a distal end of the data transmission cable 71; wherein the second interface 202 is connected with the first interface 201; the first optical module 203 is electrically connected with the image processing component 80, and is configured to obtain a first electrical signal from the image processing component 80, and convert the first electrical signal into a first optical signal; wherein the first electrical signal includes at least an image signal.

In this embodiment of this disclosure, "a proximal end" and "a distal end" referred in the endoscope imaging system, means what, an end of a component, which end is away from the image processing device 50, is a distal end of said component, and an end of said component, which end is close to the image processing device 50, is a proximal end of said component.

The data transmission cable 71 further includes at least one optical fibre 204, wherein a distal end of the optical fibre 204 is connected with the first optical module 203, wherein the optical fibre 204 is configured to transmit the first optical signal generated by the first optical module 203 to a proximal end of the optical fibre 204.

The data transmission cable 71 further includes an electrical connector 205 and a second optical module 206 which are arranged at a proximal end of the data transmission cable 71, the second optical module 206 is connected with the proximal end of the optical fibre 204 for obtaining a first optical signal transmitted by the optical fibre 204 and converting the first optical signal into a second electrical signal; wherein the second electrical signal includes at least an image signal. The electrical connector 205 is configured to be pluggable to the image processing device 50, so as to transmit the second electrical signal to the image processing device 50. In this embodiment, the electrical connector 205 is pluggable to the outside of the image processing device 50.

In this embodiment, the first optical module 203 may include an electro-optic conversion module, which is configured to convert an electrical signal into an optical signal, so as to perform optical transmission of a signal through the optical fibre 204; the second optical module 206 may include a photo-electric conversion module, which is configured to convert an optical signal transmitted by the optical fibre 204 into an electrical signal, so as to transmit the electrical signal to the image processing device 50 through the electrical connector 205.

In this embodiment of this disclosure, the first optical module 203 and the second optical module 206 are respectively arranged at both ends of the data transmission cable 71, so that optical transmission of the endoscope image signal may be implemented, and a specific solution is provided for high-speed transmission of a large data volume. The electrical connector 205 is pluggable to the image processing device 50, such that the operation portion and the data transmission cable are ensured to be cleaned and disinfected together after being unplugged. In addition, the data transmission cable 71 is connected with the image processing device 50 through the electrical connector 205, which avoids the problems of complex manufacturing process and low data transmission reliability, when an optical connector is used for connection.

In an embodiment, the data transmission cable 71 further includes a power line 210, one end of the power line 210 is connected with a power supply of the image processing device 50 through the electrical connector 205, and the other end of the power line 210 is connected with the image processing component 80 and the first optical module 203, so as to supply electric power to the image processing component 80 and the first optical module 203.

In an embodiment, the data transmission cable 71 further includes at least one control signal line 210 (in FIG. 2, the power line is not distinguished from the control signal line, and in this embodiment of this disclosure, a same reference number is used for illustration), one end of the control signal line 210 is connected with the image processing device 50 through the electrical connector 205, and the other end of the control signal line 210 is connected with the image processing component 80, so as to transmit the control electrical signal unidirectionally or bidirectionally between the image processing component 80 and the image processing device 50.

In this embodiment, the control signal includes signal(s) from physical key(s) of the operation portion 40, for example, control signal(s) of key(s) for such as brightness adjustment and focusing adjustment of the endoscope image. In some embodiments, the control signal line 210 needs to implement bidirectional transmission of the control electrical signal, for example, to transmit some control signals in the image processing device 50 to the operation portion 40, wherein these control signals may include, for example, a driving signal for focusing, which adjusts optical lens in the operation portion 40 to focus the endoscope image.

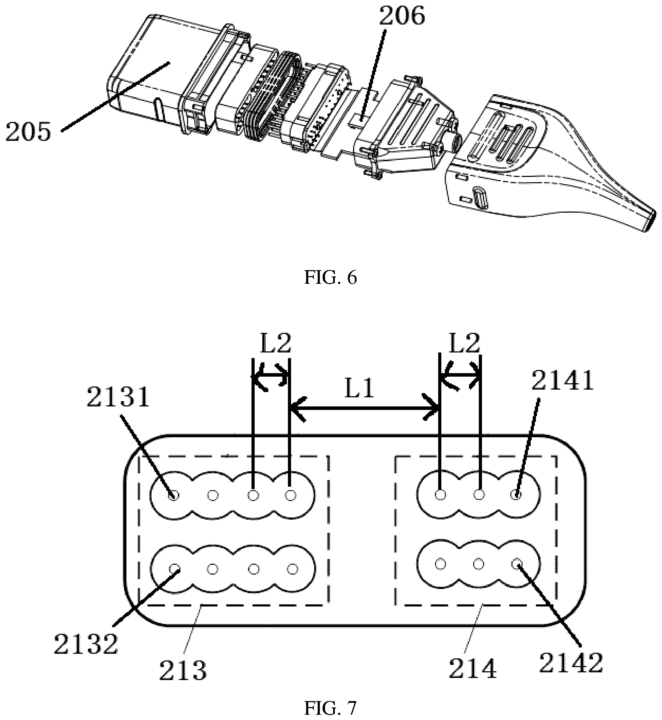

With reference to FIG. 7, in an embodiment, the electrical connector 205 includes a plurality of electrical signal terminals 208; when the electrical connector 205 is plugged to the image processing device 50, the plurality of electrical signal terminals 208 are electrically connected with the image processing device 205, so as to transmit the second electrical signal to the image processing device 50, transmit a control electrical signal between the image processing component 80 and the image processing device 50, and connect one end of the power line 210 to the power supply of the image processing device 50.

In an embodiment, as shown in FIG. 2, the image processing device 50 is provided with another electrical connector 207 which corresponds to the electrical connector 205, so as to be plugged and matched with the electrical connector 205; wherein the electrical connectors of the data transmission cable 71 and of the image processing device 50 are a pair of male plug and a female socket.

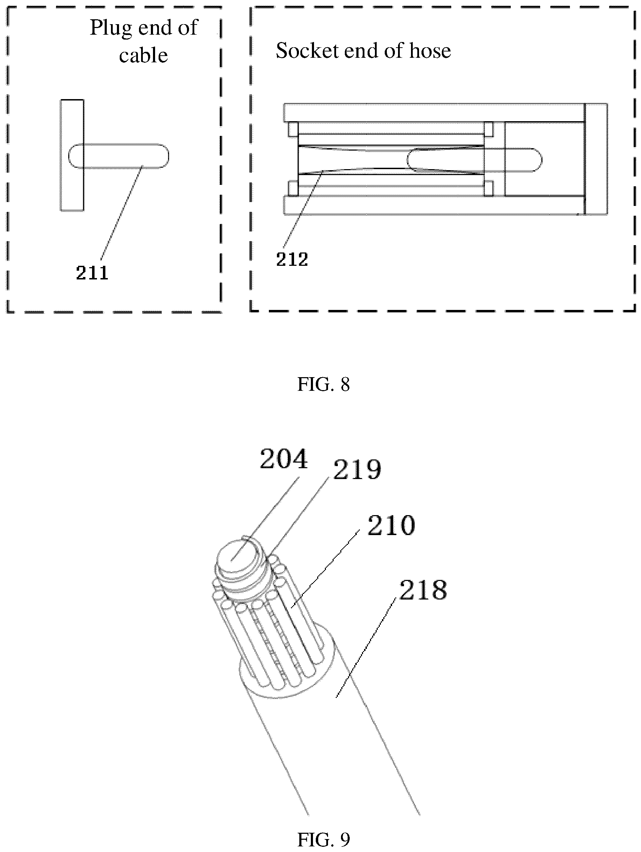

As shown in FIG. 8, in an embodiment, the electrical signal terminal 208 is a wire spring pin 211, which is configured to be matched with a wire spring jack 212 at the end of the image processing device 50. The connection mode of wire spring terminal has characteristics of multi-point contact, small insertion and extraction force and high reliability, and satisfies a requirement of the endoscope for plugging and unplugging life of the data transmission cable 71.

An electrical signal terminal is in contact connection with an terminal in the image processing device by using an elastic metal member (including but not limited to a hyperboloid wire spring, a through spring and other structures), which is higher in reliability, lower in processing difficulty and controllable in cost.

The applicant of this disclosure filed on December 31, 2021, an International Patent Application with an application No. PCT/CN2021/143064, describing an electrical connector for an endoscope imaging system, which adopts a solution of a wire spring pin and a wire spring jack, so as to improve the reliability of the electrical connector, the content of which is incorporated herein by reference in its entirety, through claiming priority.

In an embodiment, a hole of the jack is further provided with a prismatic structure shown in FIG. 7 (the pin is recessed and is lower than other external structures), thereby increasing a positioning for the plug of the data transmission cable 71 and the jack of the image processing device 50, preventing the pin from being bent due to the deviation of a plugged position of the plug, and protecting the pin from being collided by other external instruments.

In an embodiment, the plurality of electrical signal terminals 208 include a first-type terminal and a second-type terminal, a creepage distance between the first-type terminal and the second-type terminal is not less than 2.5 mm, and an air gap between the first-type terminal and the second-type terminal is not less than 1.5 mm. In this embodiment, the first-type terminal includes a connection terminal of a signal (for example, an image signal), which signal is in optical transmission inside the data transmission cable 71, and the second-type terminal includes a connection terminal of a signal (for example, a control signal), which signal is in electrical transmission inside the data transmission cable 71. Since the signal in optical transmission is usually a high-speed signal with a large data volume, and the signal in electrical transmission is usually a low-speed signal, the electrical signal terminals corresponding to the two types of signals are distinguished, and the creepage distance and the air gap between the two types of terminals are limited, so as to realize electrical isolation of the two types of signals and avoid interference.

As shown in FIG. 7, in an embodiment, the plurality of electrical signal terminals 208 include a first group of terminals 213 and a second group of terminals 214, wherein the first group of terminals 213 and the second group of terminals 214 are arranged inside two different areas of a plugged surface of the electrical connector 205 (two areas delimited by dashed lines in FIG. 7), and a distance L 1 between adjacent boundaries of the two different areas is greater than a minimum distance L 2 between any two adjacent terminals of the first group of terminals 213 or of the second group of terminals 214. The second electrical signal generated by the second optical module 206 is transmitted to the image processing device 50 through at least some terminals in the first group of terminals 213; the power line and the control signal line 210 are electrically connected with the image processing device 50 through at least some terminals in the second group of terminals 214. In this embodiment, since the second electrical signal belongs to a high-speed signal through optical transmission, the electrical signal transmitted by the power line and the control signal line 210 belongs to a low-speed signal, therefore, the connection terminals corresponding to the high-speed signal and the low-speed signal are divided into two groups, and the two groups of signals are physically isolated, so that electrical isolation of the two types of signals can be realized, and interference is avoided.

In an embodiment, in addition to the terminal for transmitting the image signal, the first group of terminals 213 further includes a first power supply terminal 2131 for connecting the power supply of the image processing device 50, so as to supply power to the second optical module 206; the second group of terminals 214 further includes a second power supply terminal 2141 for connecting the power line 210, so as to connect one end of the power line 210 to the power supply of the image processing device 50.

In an embodiment, the first group of terminals 213 further includes a first grounding terminal 2132 for connecting a designated part of a device, which device transmits the second electrical signal, to a ground potential at the image processing device 50, so as to realize protective grounding. For example, the device, which transmits the second electrical signal, includes an associated circuit board, and the first grounding terminal 2132 is configured to connect GND on said circuit board to the ground potential at the image processing device 50. The second group of terminals 214 further includes a second ground terminal 2142 for connecting a designated part of a device, which device transmits the control electrical signal, to the ground potential at the image processing device 50, so as to realize protective grounding. For example, the device, which transmits the control electrical signal includes an associated circuit board, and the second ground terminal 2142 is configured to connect GND on said circuit board to the ground potential at the image processing device 50.

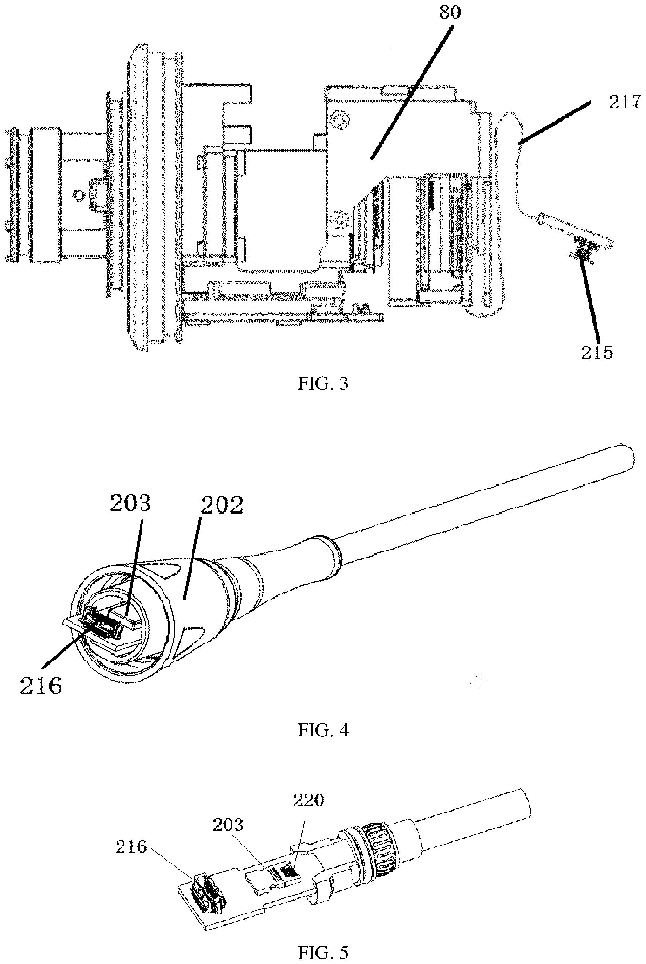

As shown in FIGS. 3-5, in an embodiment, the image processing component 80 includes a first board-to-board connector 215, the data transmission cable 71 includes a second board-to-board connector 216 which corresponds to the first board-to-board connector 215, and electrical connection between the first optical module 203 and the image processing component 80 are realized after the first board-to-board connector 215 and the second board-to-board connector 216 are plugged with each other. It should be noted that, the image processing component 80 should be considered as including: an image processing board card inside the operation portion 40, a power board, an image sensor board, a control circuit board and other element(s). The signals output by these elements may be centrally transmitted to the data transmission cable 71 through the first board-to-board connector 215 and the second board-to-board connector 216, or the image processing component 80 obtains signals from the image processing device 50 through the first board-to-board connector 215 and the second board-to-board connector 216.

In one embodiment, the first board-to-board connector 215 is electrically connected with other component(s) of the image processing component 80 through a flexible circuit board 217, and the first board-to-board connector 215 is used as a free end, so as to enable the first board-to-board connector 215 to be plugged to or unplugged from the second board-to-board connector 216. Generally, the operation portion 40 and the data transmission cable 71 are two separate components during production and assembly, and the two components need to be assembled through the first interface 201 and the second interface 202, and the first board-to-board connector 215 is connected with the image processing component 80 through the flexible circuit board 217, and serves as a free end, which may be more convenient to assemble.

In another embodiment, the second board-to-board connector 216 is electrically connected with the first optical module 203 through a flexible circuit board, and the second board-to-board connector 216 serves as a free end, so as to allow the second board-to-board connector 216 to be plugged to or unplugged from the first board-to-board connector 215.

It can be seen that one or both of the first board-to-board connector 215 and the second board-to-board connector 216 is(are) used as free end(s), and this may be selected according to actual requirements. As long as one of them is guaranteed as a free end, the purpose of convenient assembly can be achieved.

In an embodiment, the first electrical signal further includes a control signal, and the second electrical signal further includes a control signal. In this embodiment, the control signal may also include signal(s) from physical key(s) of the operation portion 40, and these control signals are transmitted to the image processing device 50 in an optical transmission manner through the optical fibre 204. In some embodiments, the control signal may all be in an optical transmission manner, or may all be in an electrical transmission manner, or may be partially in an optical transmission manner and partially in an electrical transmission manner.

In an embodiment, the first optical module 203 is further configured to respectively convert the image signal and the control signal in the first electrical signal into an optical signal, and transmit the optical signal through the optical fibre 204 in a time-division or frequency-division manner. Because the image signal and the control signal belong to two different types of signals, in order to distinguish the two types of signals in the optical transmission process and avoid mutual interference, the two types of signals may be transmitted in a time-division or frequency-division manner. Of course, in another embodiment, the two types of signals can be fused and transmitted together without separate transmission in a time-division or frequency-division manner.

In an embodiment, the second optical module 206 is further configured to obtain a third electrical signal from the image processing device 50 through the electrical connector 205, and convert the third electrical signal into a second optical signal; the optical fibre 204 is further configured to transmit the second optical signal to a distal end of the optical fibre 204; the first optical module 203 is further configured to obtain a second optical signal, and convert the second optical signal into a fourth electrical signal, and transmit the fourth electrical signal to the image processing component 80. In this embodiment, the first optical module 203 further includes a photo-electric conversion module, and the second optical module 206 further includes an electro-optic conversion module. In this embodiment, the data transmission cable 71 may implement bidirectional optical transmission of signals between the image processing component 80 and the image processing device 50.

In an embodiment, in order to distinguish bidirectional optical signals in the optical transmission process and avoid mutual interference, the first optical module 203 and the second optical module 206 are configured to respectively transmit the first optical signal and the second optical signal through the optical fibre 204 in a time-division or frequency-division manner.

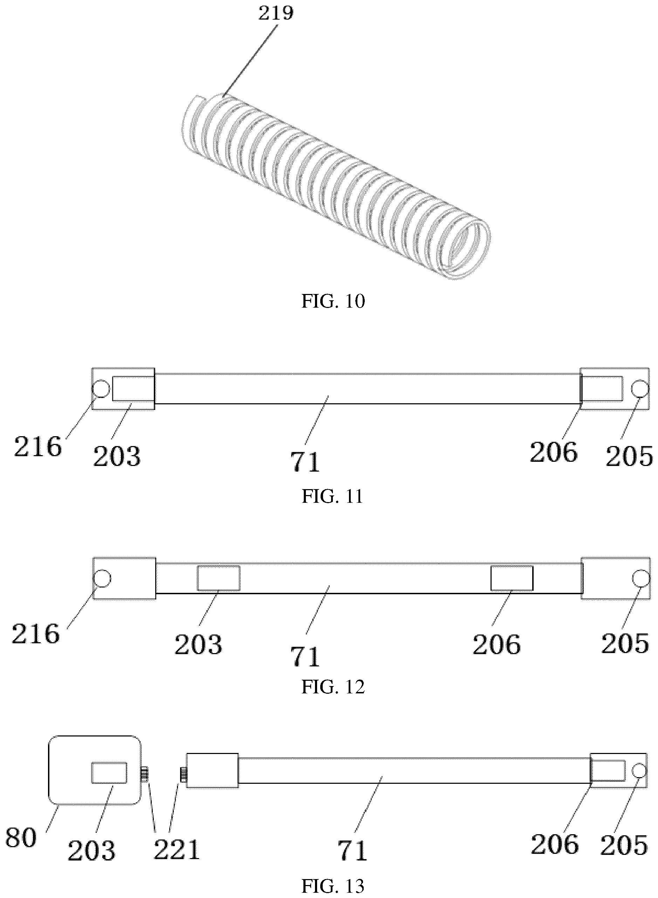

As shown in FIG. 9, in an embodiment, the data transmission cable 71 further includes an outer protective sleeve 218 and a spiral tube 219, wherein the spiral tube 219 is arranged between the optical fibre 204 and the outer protective sleeve 218, and the spiral tube 219 wraps around the optical fibre 204. The outer protective sleeve 218 is arranged at an outermost layer, and wraps around the optical fibre 204 and the spiral tube 219. As shown in FIG. 10, it is a structural diagram of the spiral tube 219 in this embodiment. Since the data transmission cable 71 is a flexible cable, it is often curved and even bent at a large angle during use. In this embodiment, the spiral tube 219 is provided to better protect the optical fibre 204, so as to prevent the optical fibre 204 from being broken in the bending process of the data transmission cable 71, thereby improving a rolling resistance and a bending resistance of the data transmission cable 71. As shown in FIG. 5, the first optical module 203 is provided with one or more optical fibre connection ports 220 for connecting optical fibers 204 inside the data transmission cable 71

In an embodiment, the power line and the control signal line 210 may be arranged outside the spiral tube 219.

The applicant of this disclosure filed on December 31, 2021, a Chinese patent application with an application No. 202111672585.8, describing a data transmission cable for an endoscope imaging system, which adopts a protection structure of a spiral tube to provide a high-reliability cable, the content of which is incorporated herein by reference in its entirety, through claiming priority.

In an embodiment, as shown in FIG. 4, the second interface 202 and the first interface 201 are in sleeve connection by threads. The second interface 202 may be sleeved at a head end of the data transmission cable 71, which end may be a free end. After the operation portion 40 is connected with the internal circuit element at the head end of the data transmission cable 71, the second interface 202 is threaded into the first interface 201.

In some embodiments, as shown in FIG. 11, two ends of the data transmission cable 71 are usually hard connection ends, so as to facilitate an arrangement of electronic elements inside the data transmission cable 71. That is, in these embodiments, the second board-to-board connector 216, the first optical module 203, and the associated circuit board card thereof are arranged inside the hard component at the distal end of the data transmission cable 71; the electrical connector 205, the second optical module 206, and the associated circuit board card thereof are arranged inside the hard component at the proximal end of the data transmission cable 71. The hard component may be a hard housing.

In some other embodiments, as shown in FIG. 12, the second board-to-board connector 216 and the associated circuit board card thereof are arranged inside a hard component at the distal end of the data transmission cable 71, and the first optical module 203 is arranged at a flexible portion at the distal end of the data transmission cable 71. The electrical connector 205 and the associated circuit board card thereof are arranged inside a hard component at the proximal end of the data transmission cable 71; the second optical module 206 is arranged at a flexible portion at the proximal end of the data transmission cable 71.

As shown in FIG. 13, an embodiment of this disclosure further provides another endoscope apparatus, which is similar to a structure of the endoscope apparatus provided in the foregoing embodiment, and the difference lies in that the optical fibre 204 arranged inside the data transmission cable 71 in the operation portion 40 is connected with the first optical module 203 through an optical connector 221, wherein the optical connector 221 is configured to transmit the optical signal generated by the first optical module 203 to the optical fibre 204. In this embodiment, as the optical connector 221 is arranged between the operation portion 40 and the data transmission cable 71, and normally, after being assembled together, the user rarely disassemble the operation portion 40 and the data transmission cable 71, and both of the operation portion 40 and the data transmission cable 71 are used together and even cleaned and sterilized together. Therefore, the optical connector 221 is arranged inside this position, which does not bring all the problems of data transmission between the data transmission cable 71 and the image processing device 50 mentioned above by using the optical connector. In these embodiments, other structures and working principles of the endoscope apparatus are the same as those of FIGS. 1-12, and details are not described herein again.

Referring also to FIGS. 1-12, an embodiment of this disclosure further provides an endoscope data transmission cable 71, which includes a second interface 202 and a first light module 203, which are arranged at a distal end of the endoscope data transmission cable 71; the second interface 202 is connected with the first interface 201 of the operation portion 40 of the endoscope apparatus; the first optical module 203 is electrically connected with the image processing component 80 of the operation portion 40, so as to obtain a first electrical signal from the image processing component 80, and convert the first electrical signal into a first optical signal; wherein the first electrical signal at least includes an image signal of a region of a patient to be observed, which signal is outputted by the image processing component 80.

The endoscope data transmission cable 71 further includes at least one optical fibre 204, and a distal end of the optical fibre 204 is connected with the first optical module 203, wherein the optical fibre 204 is configured to transmit the first optical signal which is generated by the first optical module 203 to a proximal end of the optical fibre 204.

The endoscope data transmission cable 71 further includes an electrical connector 205 and a second optical module 206, which are arranged at a proximal end of the endoscope data transmission cable 71; wherein the second optical module 206 is connected with the proximal end of the optical fibre 204, and is configured to obtain the first optical signal transmitted by the optical fibre 204 and convert the first optical signal into a second electrical signal; the second electrical signal includes at least the image signal; the electrical connector 205 is configured to be pluggable to an image processing device 50, so as to transmit the second electrical signal to the image processing device 50.

In these embodiments, the structure and working principle of the data transmission cable 71 are the same as those of FIGS. 1-12, which are not repeated here.

The above are only preferred embodiments of this disclosure and are not intended to limit this disclosure, and any modifications, equivalent replacements and improvements made within the spirit and principle of this disclosure shall fall within the protection scope of this disclosure.

## Claims

1. An endoscope apparatus, **characterized in that**, comprising:
an operation portion, which comprises a housing with a shape that is capable of being gripped, and an image processing component which is arranged inside the housing; wherein the image processing component is configured to at least output an image signal of a region of a patient to be observed; a proximal end of the operation portion is provided with a first interface; and
a data transmission cable, which comprises a second interface and a first optical module, which are arranged at a distal end of the data transmission cable; wherein the second interface is connected with the first interface; the first optical module is electrically connected with the image processing component, and is configured to obtain a first electrical signal from the image processing component, and convert the first electrical signal into a first optical signal; wherein the first electrical signal at least comprises the image signal;
the data transmission cable further comprises at least one optical fibre, wherein a distal end of the optical fibre is connected with the first optical module, the optical fibre is configured to transmit the first optical signal which is generated by the first optical module to a proximal end of the optical fibre;
the data transmission cable further comprises an electrical connector and a second optical module, which are arranged at a proximal end of the data transmission cable; wherein the second optical module is connected with the proximal end of the optical fibre, and is configured to obtain the first optical signal which is transmitted by the optical fibre, and convert the first optical signal into a second electrical signal; wherein the second electrical signal at least comprises the image signal; wherein the electrical connector is configured to be pluggable to an image processing device, so as to transmit the second electrical signal to the image processing device.

2. The endoscope apparatus according to claim 1, **characterized in that**, the data transmission cable further comprises a power line, wherein one end of the power line is connected with a power supply of the image processing device through the electrical connector, and the other end of the power line is connected with the image processing component and the first optical module, so as to supply power to the image processing component and the first optical module.

3. The endoscope apparatus according to claim 2, **characterized in that**, the data transmission cable further comprises at least one control signal line, wherein one end of the control signal line is connected with the image processing device through the electrical connector, and the other end of the control signal line is connected with the image processing component, so as to transmit a control electrical signal unidirectionally or bidirectionally between the image processing component and the image processing device.

4. The endoscope apparatus according to claim 3, **characterized in that**, the electrical connector comprises a plurality of electrical signal terminals; when the electrical connector is plugged to the image processing device, the plurality of electrical signal terminals are electrically connected with the image processing device, so as to transmit the second electrical signal to the image processing device, transmit the control electrical signal between the image processing component and the image processing device, and connect the one end of the power line to the power supply of the image processing device.

5. The endoscope apparatus according to claim 4, **characterized in that**, the plurality of electrical signal terminals comprise a first-type terminal and a second-type terminal, wherein a creepage distance between the first-type terminal and the second-type terminal is not less than 2.5 mm, and an air gap between the first-type terminal and the second-type terminal is not less than 1.5 mm.

6. The endoscope apparatus according to claim 4, **characterized in that**, the plurality of electrical signal terminals comprise a first group of terminals and a second group of terminals, the first group of terminals and the second group of terminals are arranged inside two different areas of a plugged surface of the electrical connector, and a distance between adjacent boundaries of the two different areas is greater than a minimum distance between any two adjacent terminals of the first group of terminals or of the second group of terminals; wherein the second electrical signal, which is generated by the second optical module, is transmitted to the image processing device, through at least some terminals in the first group of terminals; the power line and the control signal line are electrically connected with the image processing device through at least some terminals in the second group of terminals.

7. The endoscope apparatus according to claim 6, **characterized in that**, the first group of terminals comprise a first power supply terminal which is connected with the power supply of the image processing device, so as to supply power to the second optical module; the second group of terminals comprise a second power supply terminal which is connected with the power line, so as to connect the one end of the power line to the power supply of the image processing device.

8. The endoscope apparatus according to claim 6, **characterized in that**, the first group of terminals comprise a first ground terminal which is configured to connect a designated part of a device, which device transmits the second electrical signal, to a ground potential at the image processing device, so as to realize ground protection; the second group of terminals comprise a second ground terminal which is configured to connect a designated part of a device, which device transmits the control electrical signal, to the ground potential at the image processing device, so as to realize ground protection.

9. The endoscope apparatus according to claim 4, **characterized in that**, the electrical signal terminal is a wire spring pin, which is capable of being plugged to and matched with a wire spring jack of the image processing device.

10. The endoscope apparatus according to claim 1, **characterized in that**, the image processing component comprises a first board-to-board connector, the data transmission cable further comprises a second board-to-board connector which corresponds to the first board-to-board connector, wherein the first optical module and the image processing component are electrically connected with each other after the first board-to-board connector and the second board-to-board connector are plugged with each other.

11. The endoscope apparatus according to claim 10, **characterized in that**, the first board-to-board connector is electrically connected with other element(s) of the image processing component through a flexible circuit board, and the first board-to-board connector is used as a free end, so as to allow the first board-to-board connector to be plugged to or unplugged from the second board-to-board connector.

12. The endoscope apparatus according to claim 1, **characterized in that**, the first electrical signal further comprises a control signal, wherein the second electrical signal further comprises the control signal.

13. The endoscope apparatus according to claim 12, **characterized in that**, the control signal comprises signal(s) from physical key(s) of the operation portion.

14. The endoscope apparatus according to claim 12, **characterized in that**, the first optical module is further configured to respectively convert the image signal and the control signal in the first electrical signal into an optical signal, and transmit the optical signal through the optical fibre in a time-division or frequency-division manner.

15. The endoscope apparatus according to claim 1, **characterized in that**, the second optical module is further configured to obtain a third electrical signal from the image processing device through the electrical connector, and convert the third electrical signal into a second optical signal; the optical fibre is further configured to transmit the second optical signal to the distal end of the optical fibre; and the first optical module is further configured to obtain the second optical signal, and convert the second optical signal into a fourth electrical signal, so as to transmit the fourth electrical signal to the image processing component.

16. The endoscope apparatus according to claim 15, **characterized in that**, the first optical module and the second optical module are further configured to respectively transmit the first optical signal and the second optical signal through the optical fibre in a time-division or frequency-division manner.

17. The endoscope apparatus according to claim 1, **characterized in that**, the data transmission cable further comprises an outer protective sleeve and a spiral tube, wherein the spiral tube is arranged between the optical fibre and the outer protective sleeve, and the spiral tube wraps around the optical fibre; the outer protective sleeve is arranged at an outermost layer, and wraps around the spiral tube and the optical fibre.

18. The endoscope apparatus according to claim 1, **characterized in that**, the second interface is threaded into the first interface.

19. An endoscope apparatus, **characterized in that**, comprising:
an operation portion, which comprises a housing with a shape that is capable of being gripped, and an image processing component which is arranged inside the housing; wherein the image processing component is configured to at least output an image signal of a region of a patient to be observed; a proximal end of the operation portion is provided with a first interface;
the operation portion further comprises a first optical module; wherein the first optical module is electrically connected with the image processing component, and is configured to obtain a first electrical signal from the image processing component, and convert the first electrical signal into a first optical signal, wherein the first electrical signal at least comprises the image signal; and
a data transmission cable, which comprises a second interface that is arranged at a distal end of the data transmission cable, wherein the second interface is connected with the first interface;
the data transmission cable further comprises at least one optical fibre, wherein a distal end of the optical fibre is connected with the first optical module through an optical connector; wherein the optical fibre is configured to obtain the first optical signal from the first optical module and transmit the first optical signal to a proximal end of the optical fibre;
the data transmission cable further comprises an electrical connector and a second optical module, which are arranged at a proximal end of the data transmission cable; wherein the second optical module is connected with the proximal end of the optical fibre, and is configured to obtain the first optical signal which is transmitted by the optical fibre, and convert the first optical signal into a second electrical signal; wherein the second electrical signal at least comprises the image signal; wherein the electrical connector is configured to be pluggable to an image processing device, so as to transmit the second electrical signal to the image processing device.

20. The endoscope apparatus according to claim 19, **characterized in that**, the data transmission cable further comprises a power line, wherein one end of the power line is connected with a power supply of the image processing device through the electrical connector, and the other end of the power line is connected with the image processing component and the first optical module, so as to supply power to the image processing component and the first optical module.

21. The endoscope apparatus according to claim 20, **characterized in that**, the data transmission cable further comprises at least one control signal line, wherein one end of the control signal line is connected with the image processing device through the electrical connector, and the other end of the control signal line is connected with the image processing component, so as to transmit a control electrical signal unidirectionally or bidirectionally between the image processing component and the image processing device.

22. The endoscope apparatus according to claim 21, **characterized in that**, the electrical connector comprises a plurality of electrical signal terminals; when the electrical connector is plugged to the image processing device, the plurality of electrical signal terminals are electrically connected with the image processing device, so as to transmit the second electrical signal to the image processing device, transmit the control electrical signal between the image processing component and the image processing device, and connect the one end of the power line to the power supply of the image processing device.

23. The endoscope apparatus according to claim 22, **characterized in that**, the plurality of electrical signal terminals comprise a first-type terminal and a second-type terminal, wherein a creepage distance between the first-type terminal and the second-type terminal is not less than 2.5 mm, and an air gap between the first-type terminal and the second-type terminal is not less than 1.5 mm.

24. The endoscope apparatus according to claim 22, **characterized in that**, the plurality of electrical signal terminals comprise a first group of terminals and a second group of terminals, the first group of terminals and the second group of terminals are arranged inside two different areas of a plugged surface of the electrical connector, and a distance between adjacent boundaries of the two different areas is greater than a minimum distance between any two adjacent terminals of the first group of terminals or of the second group of terminals; wherein the second electrical signal, which is generated by the second optical module, is transmitted to the image processing device, through at least some terminals in the first group of terminals; the power line and the control signal line are electrically connected with the image processing device through at least some terminals in the second group of terminals.

25. The endoscope apparatus according to claim 24, **characterized in that**, the first group of terminals comprise a first power supply terminal which is connected with the power supply of the image processing device, so as to supply power to the second optical module; the second group of terminals comprise a second power supply terminal which is connected with the power line, so as to connect the one end of the power line to the power supply of the image processing device.

26. The endoscope apparatus according to claim 25, **characterized in that**, the first group of terminals comprise a first ground terminal which is configured to connect a designated part of a device, which device transmits the second electrical signal, to a ground potential at the image processing device, so as to realize ground protection; the second group of terminals comprise a second ground terminal which is configured to connect a designated part of a device, which device transmits the control electrical signal, to the ground potential at the image processing device, so as to realize ground protection.

27. The endoscope apparatus according to claim 22, **characterized in that**, the electrical signal terminal is a wire spring pin, which is capable of being plugged to and matched with a wire spring jack of the image processing device.

28. The endoscope apparatus according to claim 19, **characterized in that**, the first electrical signal further comprises a control signal, wherein the second electrical signal further comprises the control signal.

29. The endoscope apparatus according to claim 28, **characterized in that**, the control signal comprises signal(s) from physical key(s) of the operation portion.

30. The endoscope apparatus according to claim 28, **characterized in that**, the first optical module is further configured to respectively convert the image signal and the control signal in the first electrical signal into an optical signal, and transmit the optical signal through the optical fibre in a time-division or frequency-division manner.

31. The endoscope apparatus according to claim 19, **characterized in that**, the second optical module is further configured to obtain a third electrical signal from the image processing device through the electrical connector, and convert the third electrical signal into a second optical signal; the optical fibre is further configured to transmit the second optical signal to the distal end of the optical fibre; and the first optical module is further configured to obtain the second optical signal, and convert the second optical signal into a fourth electrical signal, so as to transmit the fourth electrical signal to the image processing component.

32. The endoscope apparatus according to claim 31, **characterized in that**, the first optical module and the second optical module are further configured to respectively transmit the first optical signal and the second optical signal through the optical fibre in a time-division or frequency-division manner.

33. The endoscope apparatus according to claim 19, **characterized in that**, the data transmission cable further comprises an outer protective sleeve and a spiral tube, wherein the spiral tube is arranged between the optical fibre and the outer protective sleeve, and the spiral tube wraps around the optical fibre; the outer protective sleeve is arranged at an outermost layer, and wraps around the spiral tube and the optical fibre.

34. The endoscope apparatus according to claim 19, **characterized in that**, the second interface is threaded into the first interface.

35. An endoscope data transmission cable, **characterized in that**, comprising a second interface and a first optical module which are arranged at a distal end of the endoscope data transmission cable; wherein the second interface is connected with a first interface of an operation portion of an endoscope apparatus, the first optical module is electrically connected with an image processing component of the operation portion, so as to obtain a first electrical signal from the image processing component, and convert the first electrical signal into a first optical signal; wherein the first electrical signal at least comprises an image signal of a region of a patient to be observed, which signal is outputted by the image processing component;
the endoscope data transmission cable further comprises at least one optical fibre, wherein a distal end of the optical fibre is connected with the first optical module; wherein the optical fibre is configured to transmit the first optical signal which is generated by the first optical module to a proximal end of the optical fibre;
the endoscope data transmission cable further comprises an electrical connector and a second optical module, which are arranged at a proximal end of the endoscope data transmission cable; wherein the second optical module is connected with the proximal end of the optical fibre, and is configured to obtain the first optical signal which is transmitted by the optical fibre, and convert the first optical signal into a second electrical signal; wherein the second electrical signal at least comprises the image signal; wherein the electrical connector is configured to be pluggable to an image processing device, so as to transmit the second electrical signal to the image processing device.

36. The endoscope data transmission cable according to claim 35, **characterized in that**, further comprising a power line, wherein one end of the power line is connected with a power supply of the image processing device through the electrical connector, and the other end of the power line is connected with the image processing component and the first optical module, so as to supply power to the image processing component and the first optical module.

37. The endoscope data transmission cable according to claim 36, **characterized in that**, further comprising at least one control signal line, wherein one end of the control signal line is connected with the image processing device through the electrical connector, and the other end of the control signal line is connected with the image processing component, so as to transmit a control electrical signal unidirectionally or bidirectionally between the image processing component and the image processing device.

38. The endoscope data transmission cable according to claim 37, **characterized in that**, the electrical connector comprises a plurality of electrical signal terminals; when the electrical connector is plugged to the image processing device, the plurality of electrical signal terminals are electrically connected with the image processing device, so as to transmit the second electrical signal to the image processing device, transmit the control electrical signal between the image processing component and the image processing device, and connect the one end of the power line to the power supply of the image processing device.

39. The endoscope data transmission cable according to claim 38, **characterized in that**, the plurality of electrical signal terminals comprise a first-type terminal and a second-type terminal, wherein a creepage distance between the first-type terminal and the second-type terminal is not less than 2.5 mm, and an air gap between the first-type terminal and the second-type terminal is not less than 1.5 mm.

40. The endoscope data transmission cable according to claim 38, **characterized in that**, the plurality of electrical signal terminals comprise a first group of terminals and a second group of terminals, the first group of terminals and the second group of terminals are arranged inside two different areas of a plugged surface of the electrical connector, and a distance between adjacent boundaries of the two different areas is greater than a minimum distance between any two adjacent terminals of the first group of terminals or of the second group of terminals; wherein the second electrical signal, which is generated by the second optical module, is transmitted to the image processing device, through at least some terminals in the first group of terminals; the power line and the control signal line are electrically connected with the image processing device through at least some terminals in the second group of terminals.

41. The endoscope data transmission cable according to claim 40, **characterized in that**, the first group of terminals comprise a first power supply terminal which is connected with the power supply of the image processing device, so as to supply power to the second optical module; the second group of terminals comprise a second power supply terminal which is connected with the power line, so as to connect the one end of the power line to the power supply of the image processing device.

42. The endoscope data transmission cable according to claim 40, **characterized in that**, the first group of terminals comprise a first ground terminal which is configured to connect a designated part of a device, which device transmits the second electrical signal, to a ground potential at the image processing device, so as to realize ground protection; the second group of terminals comprise a second ground terminal which is configured to connect a designated part of a device, which device transmits the control electrical signal, to the ground potential at the image processing device, so as to realize ground protection.

43. The endoscope data transmission cable according to claim 38, **characterized in that**, the electrical signal terminal is a wire spring pin, which is capable of being plugged to and matched with a wire spring jack of the image processing device.

44. The endoscope data transmission cable according to claim 35, **characterized in that**, the image processing component comprises a first board-to-board connector, the endoscope data transmission cable further comprises a second board-to-board connector which corresponds to the first board-to-board connector, wherein the first optical module and the image processing component are electrically connected with each other after the first board-to-board connector and the second board-to-board connector are plugged with each other.

45. The endoscope data transmission cable according to claim 44, **characterized in that**, the second board-to-board connector is electrically connected with the first optical module through a flexible circuit board, and the second board-to-board connector is used as a free end, so as to allow the second board-to-board connect to be plugged to or unplugged from the first board-to-board connector.

46. The endoscope data transmission cable according to claim 35, **characterized in that**, the first electrical signal further comprises a control signal, wherein the second electrical signal further comprises the control signal.

47. The endoscope data transmission cable according to claim 46, **characterized in that**, the control signal comprises signal(s) from physical key(s) of the operation portion.

48. The endoscope data transmission cable according to claim 46, **characterized in that**, the first optical module is further configured to respectively convert the image signal and the control signal in the first electrical signal into an optical signal, and transmit the optical signal through the optical fibre in a time-division or frequency-division manner.

49. The endoscope data transmission cable according to claim 35, **characterized in that**, the second optical module is further configured to obtain a third electrical signal from the image processing device through the electrical connector, and convert the third electrical signal into a second optical signal; the optical fibre is further configured to transmit the second optical signal to the distal end of the optical fibre; and the first optical module is further configured to obtain the second optical signal, and convert the second optical signal into a fourth electrical signal, so as to transmit the fourth electrical signal to the image processing component.

50. The endoscope data transmission cable according to claim 49, **characterized in that**, the first optical module and the second optical module are further configured to respectively transmit the first optical signal and the second optical signal through the optical fibre in a time-division or frequency-division manner.

51. The endoscope data transmission cable according to claim 35, **characterized in that**, further comprising an outer protective sleeve and a spiral tube, wherein the spiral tube is arranged between the optical fibre and the outer protective sleeve, and the spiral tube wraps around the optical fibre; the outer protective sleeve is arranged at an outermost layer, and wraps around the spiral tube and the optical fibre.

52. The endoscope data transmission cable according to claim 35, **characterized in that**, the second interface is threaded into the first interface.

53. An endoscope imaging system, **characterized in that**, comprising a light source (10), a light guide beam (20), an image processing device (50), and an endoscope apparatus according to any one of claims 1-34, wherein the light source (10) is connected with the endoscope apparatus through the light guide beam (20).
